# EUROPÄISCHE PATENTSCHRIFT

(11) **EP 2 049 179 B1**
(45) Veröffentlichungstag und Bekanntmachung des Hinweises auf die Patenterteilung: **13.12.2017**
(21) Anmeldenummer: 07787942.7
(22) Anmeldetag: 26.07.2007
(51) Int. Cl.: A61M 16/00, A61M 16/10, B01D 53/22, B01D 71/02, C01B 23/00

(54) **RÜCKHALTUNG VON EDELGASEN IM ATEMGAS BEI BEATMETEN PATIENTEN MIT HILFE VON MEMBRANSEPARATION**
RETENTION OF NOBLE GASES IN THE EXHALED AIR OF VENTILATED PATIENTS WITH THE HELP OF MEMBRANE SEPARATION
RETENUE DES GAZ RARES DANS LE GAZ RESPIRATOIRE CHEZ DES PATIENTS SOUS ASSISTANCE RESPIRATOIRE À L'AIDE D'UNE SÉPARATION PAR MEMBRANE

(30) Priorität: 26.07.2006 DE 102006034601
(43) Veröffentlichungstag der Anmeldung: 22.04.2009
(73) Patentinhaber: Schmidt, Klaus Michael, Halifax, Nova Scotia B3H 4S9 (CA)
(72) Erfinder: Schmidt, Klaus Michael, Halifax, Nova Scotia B3H 4S9 (CA)
(74) Vertreter: Weickmann & Weickmann PartmbB
(86) Internationale Anmeldenummer: PCT/EP2007/057720
(87) Internationale Veröffentlichungsnummer: WO 2008/012350

(56) Entgegenhaltungen:
- EP-A2- 0 806 215
- WO-A-2007/006377
- DE-A1- 19 646 791
- DE-C1- 19 645 223
- US-A1- 2006 130 649
- US-B1- 6 168 649

## Beschreibung

### I. Anwendungsgebiet

Die Erfindung betrifft die Aufbereitung von Gasgemischen, insbesondere die Rückhaltung von Edelgasen im Atemgas bei beatmeten Patienten. Im Speziellen betrifft die Erfindung die Verwendung einer selektiven Gastrennungsmembran zur Rückhaltung von Edelgasen im Atemgas bei beatmeten Patienten.

### II. Technischer Hintergrund

Unter den Edelgasen ist Xenon das einzige, das bei Normaldruck eine anästhetische Wirkung zeigt. Diese Wirkung wurde 1941 durch den russischen Wissenschaftler Nikolay Vasilievich Lazarev nachgewiesen.

Der narkotische Effekt des Xenons ist 1,5-mal stärker als der von Lachgas. Aufgrund seiner extrem geringen Blutlöslichkeit wird Xenon schneller abgeatmet als alle bisher bekannten Anästhetika. Zudem ist es für die Umwelt unbedenklich, da es weder ozonschädlich noch ein Treibhausgas ist, nicht brennt und zudem ungefährlich für Schwangere ist. Neben seiner anästhetischen Eigenschaft ist Xenon auch für den Schutz der Gehirnfunktionen des Patienten förderlich und eignet sich besonders für Patienten mit Herz-Kreislauf-Problemen, da während einer Xenon-Narkose die Kreislaufverhältnisse des Patienten äußerst stabil bleiben.

Aufgrund seiner Eigenschaften weist Xenon für bestimmte Indikationen deutliche Vorteile im Vergleich zu anderen Anästhetika auf. Die reinen Stoffkosten werden zwar höher ausfallen, jedoch sind die Kosten der Gesamtbehandlung aufgrund des günstigen Wirkungsprofils, der geringen Nebenwirkungen und der organschützenden Eigenschaften beim Einsatz von Xenon deutlich preiswerter.

Für den Einsatz in der Medizin wird in Zukunft die Produktion von Xenon deutlich ansteigen. Allerdings ist Xenon aufgrund seiner chemischen Eigenschaften, seiner geringen Verfügbarkeit und der damit verbundenen hohen Kosten zur Gewinnung von Xenon kein Ersatz für Lachgas und etablierte Anästhetika, sondern soll diese vielmehr ergänzen.

Eine Möglichkeit zur Kostensenkung besteht darin, das benutzte Xenon durch Recycling erneuter Verwendung zuzuführen.

Hierzu wurden im Stand der Technik verschiedene kryogene Verfahren eingesetzt.

DE 44 11 533 C1 beschreibt ein Narkosegerät mit einer Rückgewinnungsanlage für Xenon. In der Rückgewinnungsanlage wird das Expirationsgas (Ausatmungsgas) nach einer Vorreinigung komprimiert und in einen Druckbehälter eingeleitet, welcher in eine Kühlvorrichtung aufgenommen ist. Über die Kühlvorrichtung wird der Druckbehälter so weit abgekühlt, dass das rückgewonnene Xenon sich verflüssigt. Das Xenon aus dem Expirationsgas wird also in flüssigem Zustand in einem Druckbehälter gesammelt und von dort zum Patienten zurückgeführt.

WO 98/18718 beschreibt eine Apparatur und ein Verfahren zur Reinigung und Rückführung von Xenon im Anästhesiesystem, wobei das Xenon nach der Reinigung ebenfalls verflüssigt in einem kryogenen Behälter gesammelt und von dort zum Patienten zurückgeführt wird.

DE 196 35 002 A1 beschreibt ein Verfahren zur Online-Rückgewinnung von Xenon aus Narkosegas, wobei das expiratorische Atemgas mit einer Kühlfläche in Kontakt gebracht wird, die sich auf einer Temperatur unterhalb des Schmelzpunktes der abzutrennenden Komponente befindet. Das Xenon wird hierbei ausgefroren und die Verunreinigungen über das Kopfgas durch Anlegen eines Vakuums abgezogen.

WO 01/24858 A beschreibt ein System und Verfahren womit Gase, insbesondere feuchte Gase wie expiratorische Gase oder Abgase aus Anästhesiegeräten, zur Wiederaufbereitung gesammelt werden können. In der Gassammeleinrichtung wird das Gas in eine verdichtete Form wie kälteverflüssigtes, kälteverfestigtes oder komprimiertes Gas überführt.

Die genannten Systeme sind Bestandteil eines Anästhesiesystems und dienen einer direkten Rückführung des Xenons zum Patienten während der Anästhesie. Die Systeme und Verfahren sind apparativ sehr aufwendig und daher sehr kostenintensiv.

Daher wurde in letzter Zeit zunehmend nach Alternativen gesucht, die ein einfacheres und daher kostengünstiges Recycling des Xenons ermöglichen.

Besondere Bedeutung hat in diesem Zusammenhang die Gastrennung mittels einer dafür geeigneten semipermeablen Membran, einer so genannten selektiven Gastrennungsmembran.

Die Trennung von flüssigen, gasförmigen und dampfförmigen Fluidgemischen an Membranen ist in vielfältigsten Verfahrensformen bekannt. Hierbei wird mindestens eine der Komponenten des aufgebrachten Fluidgemisches von der Membran zurückgehalten und in Form eines so genannten Retentats abgeführt, während mindestens eine weitere Komponente des Fluidgemisches in der Lage ist, die Membran zu permeieren, um als Permeat auf der anderen Seite der Membran abgeführt zu werden.

Jedoch wurden erst in jüngerer Zeit Techniken entwickelt, mit denen es möglich wurde, genügend dünne und damit ausreichend durchlässige, fehlstellenfreie und mechanisch stabile Filme für die Gastrennung herzustellen. Diese Membrantypen basieren auf sehr dünnen, porenfreien, gasselektiven Filmen auf porösen Trägerschichten.

Im Stand der Technik sind beispielsweise aus EP 0 428 052 Membranen zur Gastrennung bekannt, wobei es sich um eine semipermeable Kompositmembran handelt.
Ein Verfahren zur Gasrückgewinnung, insbesondere zur Rückgewinnung von Edelgasen, aus Gasgemischen, die in Öfen zum Verschließen von Plasmabildschirmen anfallen, wobei die Abscheidung des Edelgases unter anderem durch Membrantrennung erfolgt, ist in DE 697 17 215 T2 offenbart.
DE 103 00 141 A1 beschreibt ein Verfahren zur Sauerstoffanreicherung von Luft bei gleichzeitiger Abreicherung von Kohlendioxid in einer geschlossenen oder teilweise geschlossenen Raumeinheit. Die An- bzw. Abreicherung erfolgt hierbei mittels eines Spülgasmembransystems, wobei die eingesetzten Membranen beispielsweise Aktivschichten aus Polysulfon, Polyoctylmethylsiloxan, Polyetherimid, Silikon, Ethylzellulose, Polyphenylenoxid, Polysulfon, Polycarbonat sowie Kombinationen davon aufweisen.
Aus EP 1 086 973 A2 sind Gastrennartikel aus Polyimid, wie Folien, Beschichtungen und Membranen, bekannt, die für zahlreiche Fluidtrennungsanwendungen geeignet sind.

Eine Verwendung der oben bekannten selektiven Gastrennungsmembranen zur Rückhaltung von Edelgasen im Atemgas bei beatmeten Patienten wurde nicht beschrieben.

Aus DE 196 45 223 C1 und US 2006/130649 A1 sind verschiedene Systeme mit selektiven Gastrennungsmembranen zur Rückgewinnung von im Expirationsgas von beatmeten Patienten enthaltenen Edelgasen bekannt. Diese Systeme sind jedoch apparativ sehr aufwendig und daher sehr kostenintensiv.

Aus WO 2007/006377 A1 ist die Verwendung einer selektiven Gastrennungsmembran zur Rückgewinnung von Xenon im Expirationsgas von beatmeten Patienten bekannt. Dieses Dokument gehört zum Stand der Technik nach Artikel 54(3) EPÜ.

### III. Darstellung der Erfindung

Es ist daher die Aufgabe gemäß der Erfindung, ein Verfahren bereitzustellen, welches die Rückhaltung von Edelgasen, speziell von Xenon, im Rahmen eines Gasgemisches, beispielsweise eines Beatmungsgases für Patienten, besonders einfach und unter möglichst geringen Verbrauchsverlusten an Xenon gestattet.

Diese Aufgabe wird durch die Verwendung einer mikroporösen Kohlenstofffaser-Molekularsiebmembran als selektive Gastrennungsmembran zur Rückgewinnung von im Expirationsgas von beatmeten Patienten enthaltenen Edelgasen gelöst.

Diese Aufgabe wird insbesondere durch die Verwendung einer mikroporösen Kohlenstofffaser-Molekularsiebmembran als selektive Gastrennungsmembran zur Rückgewinnung von im Atemgas von beatmeten Patienten enthaltenen Edelgasen in Verbindung mit der in der Patentanmeldung DE 10 2005 032977 beschriebenen Vorrichtung und Verfahren zur Aufbereitung von Gasgemischen gelöst. Durch die Verwendung einer solchen selektiv für Edelgase permeablen Gastrennungsmembran in dem in der Patentanmeldung DE 10 2005 032977 beschriebenen Zusatzmodul für Beatmungsgeräte wird es möglich, kostengünstig Edelgase, insbesondere Xenon, als Sedierungsmittel bei der künstlichen Beatmung von Patienten einzusetzen. Durch den dadurch erreichten geringen gerätetechnischen Aufwand ist es nun möglich Beamtungsgeräte bereitzustellen, mit denen der Edelgasverbrauch durch die Rückgewinnung derselbigen auf ein Minimum reduziert werden kann.
Die zur Abtrennung der Edelgasfraktion aus der Expirationsluft verwendete Gastrennungsmembran sollte durch eine relativ geringe Permeabilitätsrate für das gewünschte Edelgas oder Edelgasgemisch gekennzeichnet sein. Zudem sollte die Gastrennungsmembran durch einen hohen Trennungsfaktor für die gewünschte aus dem Gasgemisch abzutrennende Komponente gekennzeichnet sein. Insbesondere sollte die Gastrennungsmembran so gewählt werden, dass die in der Expirationsluft enthaltene Feuchtigkeit die Trennungseigenschaften der Membran nicht beeinflusst.

Gastrennungsmembranen, welche impermeabel für die abzutrennenden Edelgase und Edelgasgemische sind, zur erfindungsgemäßen Verwendung sind an sich aus dem Stand der Technik bekannt. Beispiele für die Anwendungen derartiger Gastrennungsmembranen können in zahlreichen Veröffentlichungen, die dem Fachmann bekannt sind, gefunden werden, wie zum Beispiel im Membrane Handbook, Winston Ho und Kamalesch Sirkar, Springer 1992.

Gastrennungsmembranen zur erfindungsgemäßen Verwendung sind mikroporöse Membranen aus Kohlenstofffasern. Kohlenstofffaser-Membranen, deren Herstellung und deren Verwendung bei der Trennung von verschiedenen Gasen sind im Stand der Technik bekannt, z.B. aus US 4 685 940, GB 2 207 666 und EP 0 621 071 B1. Bevorzugt wird die Gastrennungsmembran in einem Beatmungsgerät eingesetzt, bei dem das vom Patienten ausgeatmete Beatmungs-Rückgas an die Umgebung entlassen wird (offener Kreislauf des Hauptgasgemisches). Wird dem Beatmungsgas zur Sedierung eines Patienten neben den Hauptkomponenten des Beatmungsgases eine hierzu geeignete Fraktion, etwa ein Edelgas wie Xenon zugegeben, ist es erwünscht, den nicht verbrauchten Teil der Edelgasfraktion im Kreislauf zu führen und dem Patienten wieder zu zuführen. Hierzu ist ein Selektionselement erforderlich, das die Edelgasfraktion von weiteren Fraktionen des Expirationsgases separiert, um eine Rückgewinnung des Edelgases im Expirationsgas zu ermöglichen.
Dazu ist im Gasweg des Expirationsgases, zwischen Intubationsschlauch und dem offenen Auslass für das Expirationsgas ein Zusatzmodul, welches oben genanntes Selektionselement umfasst, erforderlich. Ein derartiges Zusatzmodul zur Verwendung in einem Beatmungsgerät für Patienten ist in den Patentanmeldungen DE 10 2005 032977 und WO 2007/006377 A1 offenbart. Das Zusatzmodul umfasst als Selektionselement erfindungsgemäß die Verwendung einer selektiven, semipermeablen Gastrennmembran, welche eine mikroporöse Kohlenstofffaser-Molekularsiebmembran ist, so dass die Edelgasfraktion von den restlichen Fraktionen des Expirationsgases abgetrennt, einem neuen Beatmungsgasgemisch zugegeben, gegebenenfalls der Edelgasanteil neu eingestellt wird und das derart aufbereitete Beatmungsgas dem Patienten zugeführt wird.
Beispielsweise findet vom Expirationsschlauch aus eine Abtrennung der Edelgasfraktion mittels einer hierfür selektiv permeablen Gastrennungsmembran statt, während der Rest des Expirationsgases an die Umgebung entlassen wird. In einer Mischkammer, welche dem das Selektionselement umfassende Zusatzmodul nachgeschaltet ist, wird für das neue Beatmungsgas aus einem Reservoir über einen steuerbaren Zufuhrregler zusätzlich verbrauchtes Edelgas zugeführt. In das Reservoir kann auch die aus dem Expirationsgas abgezogene Edelgasfraktion eingespeist werden. Hierbei ist es erforderlich, dass mittels eines Sensors der Gehalt der Edelgasfraktion in dem Gasgemisch, welches dem Patienten wieder zugeführt werden soll, bestimmt wird. Dies kann entweder in der Mischkammer oder in der Zufuhrleitung, z.B. dem Intubationsschlauch, zur Mischkammer erfolgen.

## Patentansprüche

1. Verwendung einer selektiven Gastrennungsmembran zur Rückgewinnung von im Expirationsgas von beatmeten Patienten enthaltenen Edelgasen, **dadurch gekennzeichnet, dass** die Gastrennungsmembran eine mikroporöse Kohlenstofffaser-Molekularsiebmembran ist.

2. Verwendung nach Anspruch 1, **dadurch gekennzeichnet, dass** das Edelgas aus der Gruppe ausgewählt ist, bestehend aus Helium, Neon, Krypton, Argon, Xenon und Gemischen davon.

3. Verwendung nach Anspruch 2, **dadurch gekennzeichnet, dass** das Edelgas Xenon ist.

4. Verwendung nach einem der vorhergehenden Ansprüche, **dadurch gekennzeichnet, dass** die Gastrennungsmembran Teil eines Beatmungsgerätes ist.

## Claims

1. Use of a selective gas separation membrane for the recovery of expiration gases from ventilated patents, **characterized in that** the gas separation membrane is a microporous carbon molecular sieve membrane.

2. Use according to claim 1, **characterized in that** the noble gas is selected from the group consisting of helium, neon, krypton, argon, xenon and mixtures thereof.

3. Use according to claim 2, **characterized in that** the noble gas is xenon.

4. Use according to one of the preceding claims, **characterized in that** the gas separation membrane is part of a ventilator.

## Revendications

1. Utilisation d'une membrane de séparation sélective de gaz pour la récupération de gaz rares contenus dans le gaz d'expiration de patients sous assistance respiratoire, **caractérisée en ce que** la membrane de séparation de gaz est une membrane à tamis moléculaire microporeuse à fibres de carbone.

2. Utilisation selon la revendication 1, **caractérisée en ce que** le gaz rare est choisi dans le groupe consistant en hélium, néon, crypton, argon, xénon et des mélanges de ceux-ci.

3. Utilisation selon la revendication 2, **caractérisée en ce que** le gaz rare est le xénon.

4. Utilisation selon l'une des revendications précédentes, **caractérisée en ce que** la membrane de séparation de gaz fait partie d'un appareil d'assistance respiratoire.
